(19) Europäisches Patentamt
European Patent Office
Office européen des brevets

(11) Veröffentlichungsnummer: **0 269 045 B1**

(12) **EUROPÄISCHE PATENTSCHRIFT**

(45) Veröffentlichungstag der Patentschrift: **02.01.92**

(51) Int. Cl.[5]: **C07C 67/31**, C07C 69/708, C07C 69/712

(21) Anmeldenummer: **87117212.8**

(22) Anmeldetag: **23.11.87**

# (54) Verfahren zur Herstellung von veretherten 3-Hydroxyvaleriansäureestern.

(30) Priorität: **27.11.86 DE 3640595**

(43) Veröffentlichungstag der Anmeldung:
**01.06.88 Patentblatt 88/22**

(45) Bekanntmachung des Hinweises auf die Patenterteilung:
**02.01.92 Patentblatt 92/01**

(84) Benannte Vertragsstaaten:
**BE CH DE ES FR GB IT LI NL**

(56) Entgegenhaltungen:

**ARKIV FÖR KEMI, Band 12, Nr. 25, 1958, Seiten 239-246, SALO GRONOWITZ: "3-Substituted thiophenes, Preparation and metalation of 3-methoxythiophene"**

**J.ORG.CHEMISTRY 47,(1982), S. 2745-2748**

(73) Patentinhaber: **BASF Aktiengesellschaft**
**Carl-Bosch-Strasse 38**
**W-6700 Ludwigshafen(DE)**

(72) Erfinder: **Fischer, Rolf, Dr.**
**Bergstrasse 98**
**W-6900 Heidelberg(DE)**

EP 0 269 045 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Aus Arkiv för Kemi, Band 12, Nr. 25, Seiten 243 und 244, ist bekannt, daß man durch Umsetzung von 2-Pentensäuremethylester mit Methanol in Gegenwart von Natriummethylat unter Rückflußtemperatur 3-Methoxyvaleriansäuremethylester in einer Ausbeute von 55 % erhält. Das Verfahren hat den Nachteil, daß die Ausbeute zu wünschen übrig läßt und zudem wenig wohlfeile Ausgangsstoffe verwendet werden müssen.

Aus Journal of Organic Chemistry 47 (1982), Seiten 2745 - 48, ist bekannt, daß man zum Zwecke der Isomerisierung 2- und 3-Pentensäureester mit alkalischen Mitteln wie K-t-butylat, K-methylat, DBU, Triäthylamin oder N-Methylmorpholin bei erhöhter Temperatur z.B. von 28 bis 140 °C in t-Butanol behandelt. Hierbei werden je Mol Pentenester überschüssige basische Mittel, z.B. 0,22 Mol, angewendet, jedoch keine in 3-Stellung veretherten Valeriansäureester erhalten.

Es war deshalb die technische Aufgabe gestellt, ein Verfahren zur Herstellung von veretherten 3-Hydroxyvaleriansäureestern zur Verfügung zu stellen, bei dem man von leichter zugänglichen Ausgangsstoffen ausgeht und zudem höhere Ausbeuten erzielt.

Diese Aufgabe wird gelöst in einem Verfahren zur Herstellung von veretherten 3-Hydroxyvaleriansäureestern der Formel I

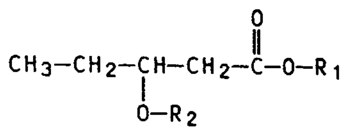

I,

in der $R_1$ und $R_2$ gleich oder verschieden sein Können und jeweils einen Alkylrest mit 1 bis 12 Kohlenstoffatomen, einen Cycloalkylrest mit 5 bis 7 Kohlenstoffatomen, einen Aralkylrest mit 7 bis 10 Kohlenstoffatomen oder einen Arylrest mit 6 bis 10 Kohlenstoffatomen bedeutet, wobei man 3-Pentensäureester der Formel II

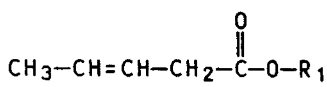

II,

in der $R_1$ die obengenannte Bedeutung hat, mit Verbindungen der Formel III

$R_3$-OH III,

in der $R_3$ die gleiche Bedeutung wie $R_2$ hat, bei erhöhter Temperatur in Gegenwart von basischen Katalysatoren umsetzt, dadurch gekennzeichnet, daß man eine Temperatur von 30 bis 95 °C und ein Molverhältnis von 3-Pentensäureester der Formel II zu basischen Katalysatoren von 1 : 0,01 bis 0,1 einhält.

Das neue Verfahren hat den Vorteil, daß man von den leicht zugänglichen 3-Pentensäureestern ausgeht. Weiter hat das neue Verfahren den Vorteil, daß es mit guten Ausbeuten und hoher Selektivität verläuft.

Das neue Verfahren ist insofern bemerkenswert als in Arkiv för Kemi, Band 12, Nr. 25, Seite 243 ausgeführt wird, daß 3-Pentensäure kein geeigneter Ausgangsstoff ist, da nur konjugierte Doppelbindungen Alkohol anlagern.

In den Ausgangsverbindungen der Formel II bezeichnet $R_1$ einen Alkylrest mit 1 bis 12 Kohlenstoffatomen, einen Cycloalkylrest mit 5 bis 7 Kohlenstoffatomen, einen Aralkylrest mit 7 bis 10 Kohlenstoffatomen oder einen Arylrest mit 6 bis 10 Kohlenstoffatomen. Geeignete Ausgangsstoffe der Formel II sind beispielsweise cis- oder trans-3-Pentensäuremethylester, 3-Pentensäureethylester, 3-Pentensäureisopropylester, 3-Pentensäurecyclohexylester, 3-Pentensäurebenzylester, 3-Pentensäurephenylester, 3-Pentensäuredodecylester. In besonders bevorzugten Ausgangsstoffen der Formel II sind $R_1$ ein Alkylrest mit 1 bis 6, insbesondere 1 bis 4 Kohlenstoffatomen, z.B. Methyl-, Ethyl-, Isopropyl-oder Butylrest. 3-Pentensäureester erhält man beispielsweise nach dem in dem US-Patent 4 550 195 beschriebenen Verfahren. Es ist auch möglich, die hierbei anfallenden Gemische aus isomeren Pentensäureestern, die neben 3-Pentensäureester 2- und/oder 4-Pentensäureester enthalten, unmittelbar für die Herstellung von veretherten 3-Hydroxyvaleriansäureestern zu verwenden, wobei 2-Pentensäureester analog umgesetzt werden, während 4-Pentensäureester nicht reagieren und abgetrennt werden.

Die Umsetzung wird mit Verbindungen der Formel III durchgeführt, in der $R_3$ einen Alkylrest mit 1 bis 12 Kohlenstoffatomen, einen Cycloalkylrest mit 5 bis 7 Kohlenstoffatomen, einen Aralkylrest mit 7 bis 10 Kohlenstoffatomen oder einen Arylrest mit 6 bis 10 Kohlenstoffatomen bezeichnet. Geeignete Verbindungen sind beispielsweise Methanol, Ethanol, n-Propanol, Isopropanol, n-Butanol, sec.-Butanol, n-Pentanol, Decanol, Cyclohexanol, Cyclopentanol, Cycloheptanol, Phenol oder Phenylethanol. In bevorzugten Verbindungen der Formel III bezeichnet $R_3$ einen Alkylrest mit 1 bis 6 Kohlenstoffatomen, insbesondere einen Alkylrest mit 1 bis 4 Kohlenstoffatomen. Vorteilhaft entsprechen die verwendeten Alkohole denjenigen der Estergruppe der Ausgangsverbindung II. Es versteht sich, daß die bevorzugten Verbindungen II und III die bevorzugten Verbindungen der Formel I ergeben.

Vorteilhaft wendet man je Mol 3-Pentensäureester der Formel II 0,5 bis 30, insbesondere 1 bis 5 mol Verbindungen der Formel III an.

Als basische Katalysatoren verwendet man vorteilhaft Alkoholate von Alkali- oder Erdalkalimetallen, ferner von Aluminium oder Titan an.

Weitere geeignete basische Katalysatoren sind Alkali- oder Erdalkaliamide oder -hydride. Geeignete Verbindungen sind beispielsweise Natriumethylat, Natriummethylat, Kaliumethylat, Magnesiumethylat, Aluminiumalkoholate oder Titanalkoholate, ferner Natriumamid, Kaliumamid, Natriumhydrid, Kaliumhydrid, Calciumhydrid. In der Regel verwendet man Alkoholate, die sich von Alkoholen mit 1 bis 4 Kohlenstoffatomen ableiten. Weitere geeignete basische Katalysatoren sind stark basische Ionenaustauscher, z.B. vernetztes Polystyrol, das quaternäre Ammoniumgruppen enthält. Besonders bevorzugt werden Alkali- und Erdalkalialkoholate oder stark basische Ionenaustauscher verwendet.

Das Molverhältnis von 3-Pentensäureestern der Formel II zu den basischen Katalysatoren beträgt von 1 : 0,01 bis 0,1. Vorteilhaft hält man Reaktionszeiten von 0,1 bis 5, insbesondere von 1 bis 2 Stunden ein.

Die Umsetzung führt man bei einer Temperatur von 30 bis 95 °C, insbesondere 40 bis 90 °C durch. In der Regel arbeitet man bei Atmosphärendruck. Es ist aber auch möglich, einen schwachen Überdruck, z.B. bis zu 10 bar, anzuwenden. Die Umsetzung kann diskontinuierlich oder kontinuierlich mit homogen gelösten oder in der Flüssigphase suspendierten basischen Katalysatoren durchgeführt werden.

Die Umsetzung führt man bei diskontinuierlicher Arbeitsweise z.B. wie folgt durch: Ein Gemisch aus 3-Pentensäureester, das noch 2- und/oder 4-Pentensäureester enthalten kann, wird mit einem Alkohol der Formel III und den vorbeschriebenen basischen Katalysatoren auf die angegebene Reaktionstemperatur erhitzt und bei dieser Temperatur bis zum möglichst vollständigen Umsatz gerührt. Vorteilhaft wird der Katalysator, z.B. durch Filtration oder Neutralisation oder Extraktion mit Wasser, abgetrennt und das Reaktionsgemisch dann durch Destillation getrennt.

Die nach dem Verfahren der Erfindung erhältlichen 3-Alkoxyvaleriansäureester eignen sich zur Herstellung von 3-Alkoxyvaleriansäuren und 3-Alkoxypentanolen.

Das Verfahren nach der Erfindung sei an folgenden Beispielen veranschaulicht.

Beispiel 1

In einem 500-ml-Dreihalskolben wurden 228,9 3-Pentensäuremethylester bei Raumtemperatur mit einer Lösung von 5,4 g Natriumethylat in 64 g Methanol (Molverhältnis 1 : 0,05 : 1) vermischt, auf 65 - 70 °C erwärmt und 4,75 Stunden bei dieser Temperatur gerührt. Nach dem Abkühlen wurde das Natriummethylat mit Eisessig neutralisiert und das Methanol bei Normaldruck weitgehend abdestilliert. Der Rückstand wurde mit Wasser gewaschen und anschließend fraktionierend destilliert. Hierbei wurden 185,6 g 3-Methoxyvaleriansäuremethylester (63 % d. Th.) von Siedepunkt 125 - 126 °C/304 mbar erhalten.

Vergleichsbeispiel 1

Eine Lösung von 0,4 g Natriumethylat in 4,7 g Methanol und 16,9 g 3-Pentensäuremethylester (Molverhältnis 1 : 0,05 : 1) wurde in einem Glasautoklaven 4 Stunden lang auf 135 °C erhitzt. Nach dieser Zeit bestand das Reaktionsgemisch zu 60 % aus einem Gemisch aus 2-Propenyliden- und 2-(1-Propenyl)-3-ethylglutarsäuredimethylester, zu 15 % aus 3-Methoxyvaleriansäuremethylester.

Beispiel 2

Eine Lösung von 5 g 3-Pentensäuremethylester und 0,12 g Natriummethylat in 50 ml Methanol

(Molverhältnis 1 : 0,05 : 28) wurde 7 Stunden lang zum Rückfluß erhitzt. Nach dieser Zeit bestand das Reaktionsgemisch laut gaschromatographischer Analyse zu 55 % aus 3-Methoxyvaleriansäuremethylester.

Beispiel 3

Ein Gemisch aus 192 g cis + trans-3-Pentensäuremethylester, 4 g 2-cis-Pentensäuremethylester und 28 g 2-trans-Pentensäuremethylester wurde mit einer Lösung von 5,4 g Natriummethylat in 64 g Methanol versetzt, auf 65 ° C erwärmt und vier Stunden bei dieser Temperatur gerührt. Nach der im Beispiel 1 beschriebenen Aufarbeitung wurden 153,6 g 3-Methoxyvaleriansäuremethylester (53 % d. Th.) erhalten.

Beispiel 4

Eine Suspension von 20 g eines stark basischen Ionenaustauschers in 32 g Methanol wurde bei 65 ° C mit 11,4 g 3-Pentensäuremethylester versetzt und 3 Stunden bei dieser Temperatur gerührt. Die gaschromatographische Analyse zeigte, daß im Reaktionsgemisch 60 % 3-Methoxyvaleriansäuremethylester enthalten waren. Nach 6 Stunden Reaktionszeit waren 65 % 3-Methoxyvaleriansäuremethylester entstanden.

Beispiel 5

156 g 3-Pentensäure-n-butylester wurden bei Raumtemperatur mit einer Lösung von 4,8 g Natriumbutanolat in 74 g n-Butanol versetzt, auf 65 ° C erwärmt und 6 Stunden bei dieser Temperatur gerührt. Das Reaktionsgemisch wurde abgekühlt, nach dem Abdestillieren des n-Butanols mit 25 ml Wasser ausgeschüttelt und über $Na_2SO_4$ getrocknet. Durch fraktionierende Destillation wurden 95 g 3-n-Butoxyvaleriansäure-n-butylester (41 % d. Th.) erhalten.

Beispiel 6

Eine Lösung von 0,3 g Natriumhydrid (80%ig) in 22,8 g 3-Pentensäuremethylester und 6,4 g Methanol wurde 4 Stunden bei 65 ° C gerührt. Die gaschromatographische Analyse zeigte, daß das Reaktionsgemisch zu 51 % aus 3-Methoxyvaleriansäuremethylester bestand.

Beispiel 7

Wurden in Beispiel 6 die 0,3 g Natriumhydrid durch 0,8 g Natriumamid (50%ig, in Toluol) ersetzt, so zeigte die gaschromatographische Analyse, daß das Reaktionsgemisch zu 63 % aus 3-Methoxyvaleriansäuremethylester bestand.

**Patentansprüche**

**1.** Verfahren zur Herstellung von veretherten 3-Hydroxyvaleriansäureestern der Formel I

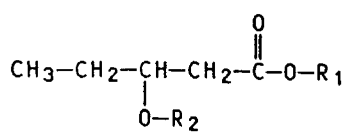

I,

in der $R_1$ und $R_2$ gleich oder verschieden sind und jeweils einen Alkylrest mit 1 bis 12 Kohlenstoffatomen, einen Cycloalkylrest mit 5 bis 7 Kohlenstoffatomen, einen Aralkylrest mit 7 bis 10 Kohlenstoffatomen oder einen Arylrest mit 6 bis 10 Kohlenstoffatomen bedeutet, wobei man 3-Pentensäureester der Formel II

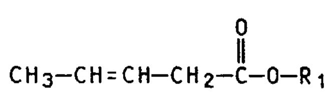

II,

in der $R_1$ die obengenannte Bedeutung hat, mit Verbindungen der Formel III

$R_3$-OH III,

in der $R_3$ die gleiche Bedeutung wie $R_2$ hat, bei erhöhter Temperatur in Gegenwart von basischen Katalysatoren umsetzt, dadurch gekennzeichnet, daß man eine Temperatur von 30 bis 95°C und ein Molverhältnis von 3-Pentensäureester der Formel II zu basischen Katalysatoren von 1 : 0,01 bis 0,1 einhält.

**2.** Verfahren nach Anspruch 1, dadurch gekennzeichnet, daß man als basische Katalysatoren Alkoholate, Amide oder Hydride von Alkali- oder Erdalkalimetallen oder stark basische Ionenaustauscher verwendet.

**3.** Verfahren nach den Ansprüchen 1 und 2, dadurch gekennzeichnet, daß man je Mol 3-Pentensäureester 0,5 bis 30 mol Verbindungen der Formel III anwendet.

**4.** Verfahren nach den Ansprüchen 1 bis 3, dadurch gekennzeichnet daß man Alkanole mit 1 bis 4 Kohlenstoffatomen verwendet.

**5.** Verfahren nach den Ansprüchen 1 bis 4, dadurch gekennzeichnet, daß man 3-Pentensäure-$C_1$-$C_4$-Alkylester verwendet.

**6.** Verfahren nach den Ansprüchen 1 bis 5, dadurch gekennzeichnet, daß man eine Temperatur von 40 bis 90°C einhält.

## Claims

**1.** A process for the preparation of an etherified 3-hydroxyvalerate of the formula I

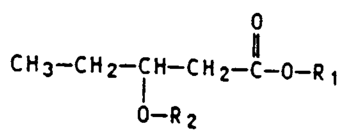

I

and where $R_1$ and $R_2$ are identical or different and are each alkyl of 1 to 12 carbon atoms, cycloalkyl of 5 to 7 carbon atoms, aralkyl of 7 to 10 carbon atoms or aryl of 6 to 10 carbon atoms, a 3-pentenoate of the formula II

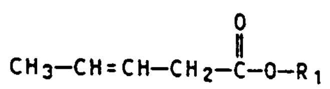

II

where $R_1$ has the above meanings, being reacted with a compound of the formula III

$R_3$-OH III

where $R_3$ has the same meanings as $R_2$, at elevated temperature in the presence of a basic catalyst, wherein a temperature of from 30 to 95°C and a molar ratio of the 3-pentenoate of the formula II to the basic catalyst of from 1:0.01 to 0.1 are maintained.

**2.** A process as claimed in claim 1, wherein the basic catalyst used is an alcoholate, amide or hydride of an alkali metal or alkaline earth metal, or a strongly basic ion exchanger.

**3.** A process as claimed in either of claims 1 and 2, wherein from 0.5 to 30 moles of the compound of the formula III are used per mole of 3-pentenoate.

**4.** A process as claimed in any of claims 1 to 3, wherein an alkanol of 1 to 4 carbon atoms is used.

**5.** A process as claimed in any of claims 1 to 4, wherein a $C_1$-$C_4$-alkyl 3-pentenoate is used.

**6.** A process as claimed in any of claims 1 to 5, wherein a temperature of from 40 to 90° C is maintained.

## Revendications

**1.** Procédé de préparation d'esters éthérifiés de l'acide 3-hydroxyvalérique, de formule I

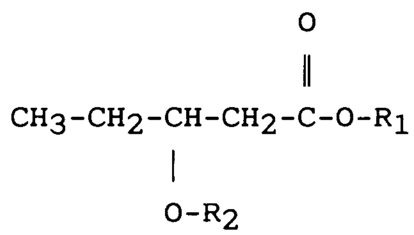

I,

dans laquelle $R_1$ et $R_2$ sont identiques ou différents et représentent chacun un radical alkyle ayant 1 à 12 atomes de carbone, un radical cycloalkyle ayant 5 à 7 atomes de carbone, un radical aralkyle ayant 7 à 10 atomes de carbone ou un radical aryle ayant 6 à 10 atomes de carbone, dans lequel on fait réagir un ester de l'acide 3-penténoïque de formule II

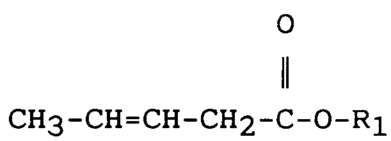

II,

dans laquelle $R_1$ a les significations ci-dessus, avec des composés de formule III

$R_3$-OH III,

dans laquelle $R_3$ a les mêmes significations que $R_2$, à haute température et en présence de catalyseurs basiques, caractérisé en ce qu'on maintient une température de 30 à 95° C et un rapport en moles de l'ester de l'acide 3-penténoïque de formule II aux catalyseurs basiques de 1:0,01 à 0,1.

**2.** Procédé selon la revendication 1, caractérisé en ce qu'on utilise comme catalyseurs basiques des alcoolates, des amides ou des hydrures de métaux alcalins ou alcalino-terreux, ou encore des échangeurs d'ions fortement basiques.

**3.** Procédé selon les revendications 1 et 2, caractérisé en ce qu'on utilise par mole d'ester de l'acide 3-penténoïque 0,5 à 30 moles de composés de formule III.

**4.** Procédé selon les revendications 1 à 3, caractérisé en ce qu'on utilise des alcanols ayant 1 à 4 atomes de carbone.

**5.** Procédé selon les revendications 1 à 4, caractérisé en ce qu'on utilise un ester alkylique en $C_1$-$C_4$ de l'acide 3-penténoïque.

**6.** Procédé selon les revendications 1 à 5, caractérisé en ce qu'on maintient une température de 40 à 90° C.